# EUROPEAN PATENT SPECIFICATION

(11) **EP 0 391 070 B1**
(45) Date of publication and mention of the grant of the patent: **19.01.1994**
(21) Application number: 90104018.8
(22) Date of filing: 01.03.1990
(51) Int. Cl.: C07C 217/62, C07C 213/06, C07C 217/04

(54) **Process for the preparation of N-methyl-3-(p-trifluoro-methylphenoxy)-3-phenyl-propylamine and their salts**
Verfahren zur Herstellung von N-Methyl-3-(p-Trifluoromethyl-phenoxy)-3-phenyl-propylamin und ihre Salze
Procédé pour la préparation de N-méthyl-3-(p-trifluoro-méthylphénoxy)-3-phényl-propylamine et leurs sels

(30) Priority: 03.03.1989 FI 891015
(43) Date of publication of application: 10.10.1990
(73) Proprietor: ORION-YHTYMÄ OY FERMION, 02101 Espoo (FI)
(72) Inventor: Kairisalo, Pekka Juhani, SF-00950 Helsinki (FI); Hukka, Petri Juhani, SF-02170 Espoo (FI); Järvinen, Anitta Hannele, SF-00960 Helsinki (FI)
(74) Representative: VOSSIUS & PARTNER

(56) References cited:
- GB-A- 2 060 618
- US-A- 4 314 081
- PATENT ABSTRACTS OF JAPAN, vol. 10, no. 239 (C-367)(2295), 19 August 1986, & JP-A-61 72736
- JOURNAL OF LABELLED COMPOUNDS AND RADIOPHARMACEUTICALS, vol. 24, no. 11, 1987, JOHN WILEY & SONS, LTD, pages 1397-1404; D. W. ROBERTSON et al, "SYNTHESIS OF 14C- AND 3H-LABELED FLUOXETINE, A SELECTIVE SEROTONIN UPTAKE INHIBITOR"

## Description

The present invention relates to a process for the preparation of N-methyl-3-(p-trifluoromethylphenoxy)-3-phenylpropylamine of formula (I), 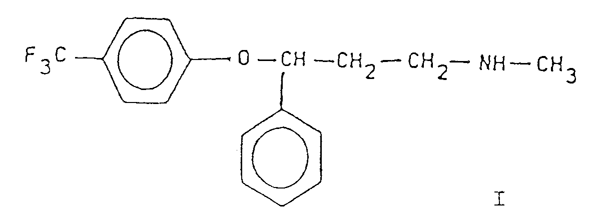
or a pharmaceutically acceptable acid addition salt thereof.

Fluoxetine, (N-methyl-3-(p-trifluoromethylphenoxy)-3-phenylpropylamine) the pharmacological effect of which is based on its inhibiting effect on serotonine (5HT) uptake in vivo and which is therefore used as an efficient antidepressant, is a previously well known compound. The preparation of Fluoxetine has thus been described earlier in American patent US 4,314,081, in British patent GB 2,060,618, and in Journal of Labelled Compounds and Radiopharmaceuticals, Vol. 24, N° 11(1987), pp. 1397-1404.

In the process for the preparation of Fluoxetine, as described in the American patent, US 4,314,081, β-dimethylaminopropiophenone hydrochloride is used as a starting compound which, after the liberation of the base, is hydrogenated with diborane, B₂H₆. The N,N-dimethyl-3-hydroxy-3-phenylpropylamine produced in the reaction is allowed to react with thionylchloride in hydrochloric acid, whereby N,N-dimethyl-3-phenyl-3-chloropropylamine is obtained. This compound is allowed to react under alkaline conditions with p-trifluoromethylphenol by refluxing the mixture for 5 days, whereby N,N-dimethyl-3-p-trifluoromethylphenoxy-3-phenylpropylamine is produced. This compound is N-demethylated with the aid of cyanobromide, whereupon N-methyl-N-cyano-3-(p-trifluoromethylphenoxy)-phenylpropylamine is obtained as a product. The N-cyano group is removed from this compound by refluxing it for 20 hours at 130 °C in a mixture of KOH-ethylene glycol. The reaction mixture is extracted with ether, and the ether phase is evaporated to dryness. The residue, i.e. Fluoxetine, can further be purified by recrystallization or be converted in a known manner into Fluoxetine hydrochloride.

The British patent, GB 2,060,618, describes two subjects the latter of which relates to the preparation of Fluoxetine. In the latter process, N-methyl-3-hydroxy-3-phenylpropylamine is used as the starting compound, which is allowed to react with 1-fluror-4-trifluoromethylbenzene in the presence of sodium hydride and with dimethylsulfoxide as a solvent. The mixture is heated to 80 °C, whereafter it is allowed to cool to room temperature. The oily residue is poured onto a mixture of ice and water, which is extracted with ether. The ether phase is dried, and the ether is distilled off in a vacuum. The residue is dissolved in ether and ether-hydrochloric acid (g) is added, whereupon the hydrochloride salt of Fluoxetine precipitates. The precipitate is filtered, washed with ether, and dried in a vacuum.

D.W. Robertson and al., Journal of Labelled Compounds and Radiopharmaceuticlas, Vol. 24, N° 11 (1987) pp. 1397-1404, describe the synthesis of ³H- and ¹⁴C-labelled fluoxetine and their brominated precursors by reacting α-(2-(dimethylamino)-ethyl)-3-bromobenzenemethanol or α-2-(methylamino)-ethyl)-benzenemethanol with sodium hydride, followed by the addition of p-chloro-benzotrifloride in N,N-dimethylacetamide. This process is very disadvantageous on an industrial scale because of the drawbacks associated with the use of sodium hydride and because of its low yields.

The JP-A-6 172 736 (cited in Patent Abstracts of Japan, Vol. 10, N° 239 ((C-367)(2295) describes the catalytic reduction of α-aminoketones with a catalyst such as Pd/C for the preparation of ephedrine derivatives.

It is the task of the present invention to provide a process for the preparation of N-methyl-3-(p-trifluoromethylphenoxy)-3-phenylpropylamine or a pharmaceutically acceptable acid addition salt thereof in a way which is mor advantageous both technically and economically, especially in a good yield and in a very pure form, starting from more easily available compounds.

Subject matter of the present invention is a process for the preparation of N-methyl-3-(p-trifluoromethylphenoxy)-3-phenyl-propylamine of formula (I), 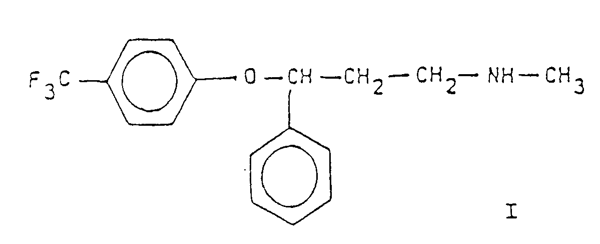
or a pharmaceutically acceptable acid addition salt thereof, which is characterized in that 2-benzoyl-N-benzyl-N-methylethylamine base of formula (II), 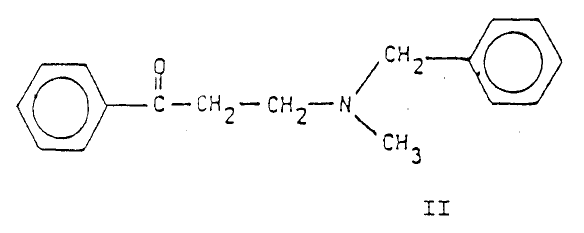
is hydrogenated catalytically, whereby 1-phenyl-3-(N-methylamino)-propane-1-ol of formula (III) is formed, 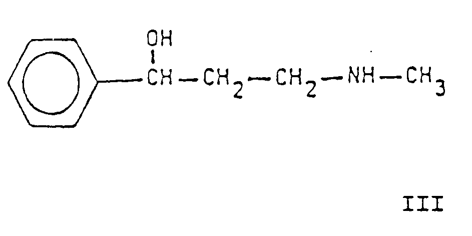
which is thereafter etherified selectively with 1-chloro-4-trifluoromethylbenzene of formula (IV), 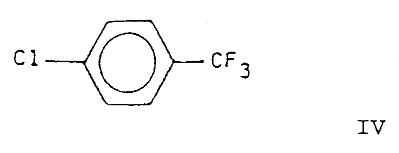
in the presence of potassium t-butoxide as base, whereby N-methyl-3-(p-trifluoro-methylphenoxy)-3-phenyl-propylamine is formed, which is optionally converted in a known manner into the acid addition salt of Fluoxetine, e.g. Fluoxetine hydrochloride.

Thus, the 2-benzoyl-N-benzyl-N-methylethylamine base (II) is first produced in a known manner, and is then hydrogenated catalytically, e.g. with the aid of Pt/C or Pd-Pt/C.

Thereafter the following measures are used in accordance with a special embodiment of this invention:

The reaction is performed at a hydrogen gas pressure of 5 bar and at a temperature of 50°C, with ethanol or ethylacetate as the solvent. The 1-phenyl-3-(N-methylamino)-propane-1-ol produced in the hydrogenation reaction is thereafter etherified selectively by using 1-chloro-4-trifluoromethylbenzene in N-methylpyrrolidone (NMP) in the presence of KJ and with potassium-t-butoxide as the base. In this way, N-methyl-3-(p-trifluoromethylphenoxy)-3-phenyl-propylamine base is obtained, and it can be converted in a known manner, with an almost theoretical yield, e.g. into the corresponding hydrochloride salt, i.e. Fluoxetine hydrochloride.

The process of the present invention has several considerable advantages over the processes described earlier. Thus, in the process described in the American patent, US 4,314,081, N,N-dimethylaminopropiophenone is used as the starting compound, on which a complicated N-demethylation has to be performed with the aid of cyanobromide at a later stage. According to the process of the present invention, 2-benzoyl-N-benzyl-N-methylethylamine is first prepared, its keto group being reduced and its N-benzyl group easily splitting off in the same reaction step, whereby the desired 1-phenyl-3-N-monomethylaminopropanol is obtained as an intermediate. By the process of the present invention, the cumbersome demethylation with the aid of cyanobromide is in this way avoided. The process of the present invention has also another considerable advantage over the process described in US 4,314,081 in that, according to the process of the present invention, 1-phenyl-3-N-methylaminopropanol is allowed to react with p-chlorobenzotrifluoride. This reaction occurs rapidly and with a good yield, whereas according to US patent 4,314,081 p-trifluoromethylphenol must be heated together with N,N-dimethyl-3-phenyl-3-chloropropylamine for as many as 5 days in order to obtain at least some N,N-dimethyl-3-p-trifluoromethylphenoxy-3-pnehylpropylamine. Moreover, the above-mentioned N-demethylation has to be performed on this intermediate. In the experiments we have performed, we have obtained by this procedure a maximum yield of 20 %, because the hydroxyl group of p-trifluoromethylphenol reacts very poorly with the chloro substituent of N,N-dimethyl-3-phenyl-3-chloropropylamine. The process according to the present invention is therefore superior to the process described in the American patent, US 4,314,081, both from a technical and an economic viewpoint.

On the other hand, in a comparison of the process of the present invention with the process described in the British patent, GB 2,060,618, it can be noted, first, that when 1-phenyl-3-N-methylaminopropanol is etherified, in accordance with the process of the invention, p-chlorobenzotrifluoride is used, the price of which is only 1/10 of the price of the p-fluorobenzotrifluoride used in the GB patent 2,060,618, but nevertheless it reacts equally well. In the process according to the British patent, the reagent used is sodium hydride, which is very prone to explode, especially when coming into contact with moisture. The reaction must therefore be performed under conditions completely devoid of water; this is very difficult to achieve on an industrial scale. In the process according to the present invention, sodium hydride can be replaced by potassium-t-butoxide, the use of which is completely safe on an industrial scale. The yield of Fluoxetine hydrochloride in the process according to the example of the British patent is only 63.4 %, whereas a yield of over 85 % is achieved by the process according to the present invention. Moreover, large quantities, i.e. about 1 mole of sodium hydride per mole of 1-phenyl-3-N-methylpropane-1-ol, are needed in the etherifying; this is hazardous in terms of occupational safety.

In the second process of the British patent there is no mention of how the 1-phenyl-3-N-monomethylaminopropanol used as the starting compound is prepared.

The process of the present invention has thus considerable advantages, both technical and economic, as well as advantages connected with occupational safety, as compared with the process described in the British patent, GB 2,060,618. The invention is best illustrated with examples.

### Example 1

### 1-phenyl-3-N-methylaminopropane-1-ol

40 g (0.158 mole) of 2-benzoyl-N-benzyl-N-methylethylamine is hydrogenated in an autoclave with the aid of 3-4 g Pt-Pd/C and with ethyl acetate as the solvent. The reaction takes place during 2 hours at a hydrogen pressure of 5 bar and at a temperature of 50 °C.
After the hydrogenation the catalyst is filtered off using a pressure filter. The ethyl acetate is distilled off from the obtained solution at normal pressure in such a way that the temperature of the mixture is at the end 130-135 °C. The residue from the distillation is allowed to cool to 70 °C, whereafter 100 ml of heptane is added dropwise thereto.

Thereafter the mixture is first cooled to 25 °C, and when the product begins to crystallize, the mixture is further cooled to 5-10 °C and the mixing is continued for one more hour.

Finally the precipitate is filtered off and washed twice with 30 ml of cold heptane. The precipitate is dried in a vacuum at 50 °C. The yield is 22.2 g of 1-phenyl-3-N-methylaminopropane-1-ol (85 % of the theoretical yield.).

### Analysis:

¹H-NMR (CDCl₃): 7.2 (multiplet 5H); 4.8 (quartet 1H); approx. 4.0 (singlet 1H); 2.7 (multiplet 2H); 2.3 (singlet 3H); 1.7 (multiplet 2H)

### Example 2

### N-methyl-3-(p-trifluoromethylphenoxy)-3-phenyl-propylamine

150 ml of N-methylpyrrolidone and 18.5 g (0.152 mole) of potassium-t-butoxide are transferred into a reaction vessel with a N₂ gas shield and are mixed for 15 minutes. 25 g (0.152 mole) of 1-phenyl-3-N-methylaminopropanol, 0.3 g of potassium iodide and 36,0 g (0.199 mole) of p-chlorobenzotrifluoride are added to the mixture, and the mixing is continued for 6 hours at 80 °C. The solution is cooled to 25 °C, and 300 ml of water is added. Thereafter the solution is extracted twice with 200 ml of toluene. The toluene layers are combined and washed five times with 100 ml of water. The toluene is dried on sodium sulfate and is evaporated to dryness.
42 g (90 % of the theoretical) of a very pure N-methyl-3-(p-trifluoromethylphenoxy)-3-phenylpropylamine base is obtained as an evaporation residue.
The base is converted in a known manner into Fluoxetine hydrochloride, with an almost quantitative yield. M.p. = 154 - 155 °C.

### Analysis:

- ¹H-NMR (CDCl₃):: 7.4 (doublet 2H); 7.3 (multiplet 5H); 6.9 (doublet 2H); 5.3 (two doublets 1 H); 2.7 (triplet 2H); 2.4 (singlet 3H); 2.2 (multiplet 1H); 2.0 (multiplet 1H); 1.4 (singlet 1H)

## Claims

1. A process for the preparation of N-methyl-3-(p-trifluoromethylphenoxy)-3-phenyl-propylamine of formula (I), 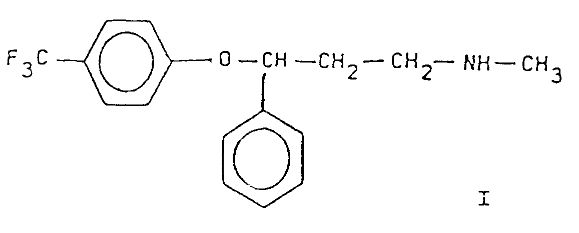 or a pharmaceutically acceptable acid addition salt thereof, characterized in that 2-benzoyl-N-benzyl-N-methylethylamine base of formula (II), 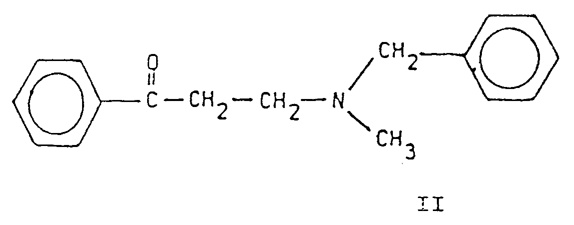 is hydrogenated catalytically, whereby 1-phenyl-3-(N-methylamino)-propane-1-ol of formula (III) is formed, 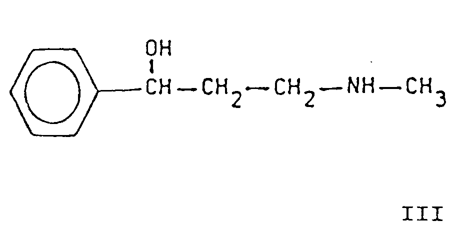 which is thereafter etherified selectively with 1-chloro-4-trifluoromethylbenzene of formula (IV), 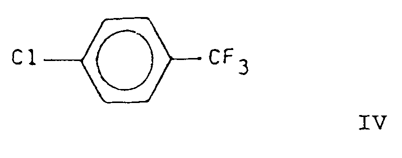 in the presence of potassium t-butoxide as base whereby N-methyl-3-(p-trifluoromethylphenoxy)-3-phenyl-propylamine is formed, which is optionally converted in a known manner into the acid addition salt of Fluoxetine, e.g. Fluoxetine hydrochloride.

2. Process of claim 1, characterized in that the hydrogenation of the compound of formula (II) is carried out in the presence of Pd/C or Pd-Pt/C catalyst.

3. Process of at least one of the claims 1 - 2, characterized in that the hydrogenation is carried out in the presence of water or an organic solvent, e.g. ethyl acetate.

4. Process of at least one of the claims 1 - 3, characterized in that the hydrogenation is carried out at a hydrogen pressure of 1-20 bar at elevated temperature, e.g. at 50°C.

5. Process of at least one of the claims 1 - 4, characterized in that the etherification of compound III is carried out in an organic solvent, e.g. N-methylpyrrolidone at elevated temperature, e.g. at 80°C.

## Patentansprüche

1. Verfahren zur Herstellung von N-Methyl-3-(p-trifluormethylphenoxy)-3-phenylpropylamin der Formel (I), 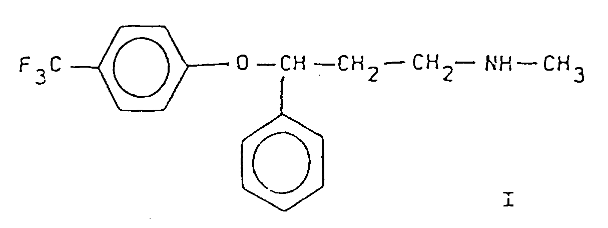 oder eines pharmazeutisch annehmbaren Säureadditionssalzes davon, dadurch gekennzeichnet, daß 2-Benzoyl-N-benzyl-N-methylethylamin-Base der Formel (II), 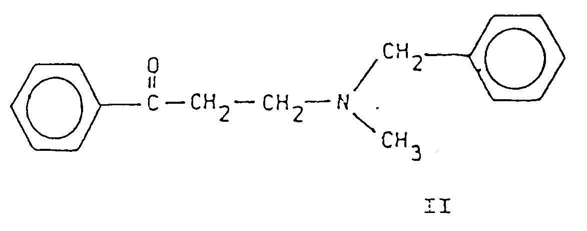 katalytisch hydriert wird, wobei 1-Phenyl-3-(N-methylamino)-propan-1-ol der Formel (III), 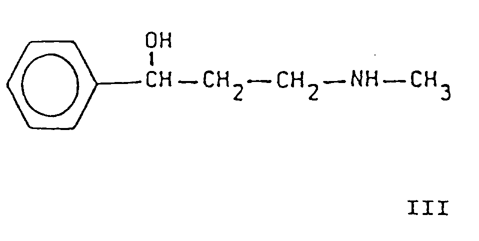 gebildet wird, das anschließend selektiv mit 1-Chlor-4-trifluormethylbenzol der Formel (IV), 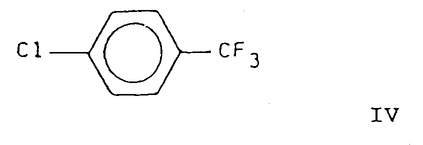 in Gegenwart von Kalium-t-butylat als Base verethert wird, wobei N-Methyl-3-(p-trifluormethylphenoxy)-3-phenylpropylamin gebildet wird, das wahlweise auf eine bekannte Weise in das Säureadditionssalz von Fluoxetin, beispielsweise Fluoxetin-Hydrochlorid, überführt wird.

2. Verfahren nach Anspruch 1, dadurch gekennzeichnet, daß die Hydrierung der Verbindung der Formel (II) in Gegenwart eines Pd/C- oder Pd-Pt/C-Katalysators durchgeführt wird.

3. Verfahren nach wenigstens einem der Ansprüche 1 - 2, dadurch gekennzeichnet, daß die Hydrierung in Gegenwart von Wasser oder eines organischen Lösungsmittels, z. B. Ethylacetat, durchgeführt wird.

4. Verfahren nach wenigstens einem der Ansprüche 1 - 3, dadurch gekennzeichnet, daß die Hydrierung bei einem Wasserstoffdruck von 1-20 bar bei erhöhter Temperatur, z. B. bei 50°C, durchgeführt wird.

5. Verfahren nach wenigstens einem der Ansprüche 1 - 4, dadurch gekennzeichnet, daß die Veretherung der Verbindung III in einem organischen Lösungsmittel, z. B. N-Methylpyrrolidon, bei erhöhter Temperatur, z. B. bei 80°C, durchgeführt wird.

## Revendications

1. Procédé de préparation de N-méthyl-3-(p-trifluorométhylphénoxy)-3-phényl-propylamine de formule (I) 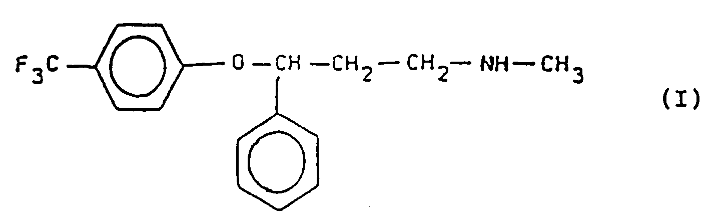 ou d'un sel d'addition d'acides pharmaceutiquement acceptable de celle-ci, caractérisé en ce qu'une base de 2-benzoyl-N-benzyl-N-méthyléthylamine de formule (II) 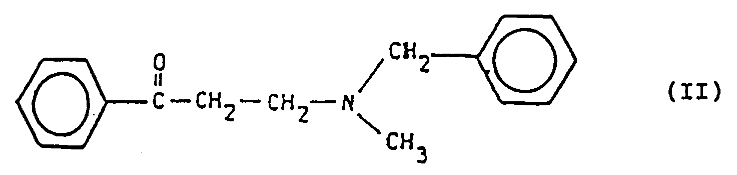 est hydrogénée catalytiquement, grâce à quoi un 1-phényl-3-(N-méthylamino)propane-1-ol de formule (III) est formé 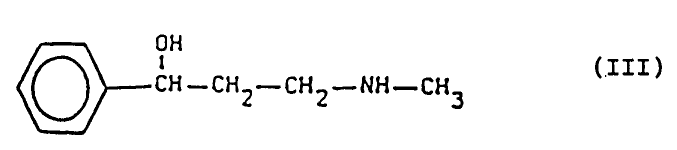 qui est ensuite éthérifié de manière sélective avec du 1-chloro-4-trifluorométhylbenzène de formule (IV) 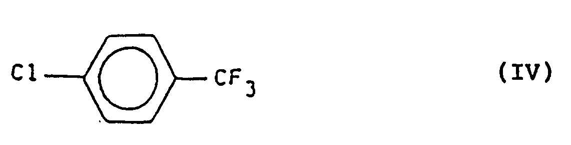 en présence de t-butylate de potassium, comme base, grâce à quoi une N-méthyl-3-(p-trifluorométhylphénoxy)-3-phénylpropylamine est formée, qu'on transforme éventuellement, d'une manière connue, en le sel d'addition d'acides de Fluoxetine, par exemple le chlorhydrate de Fluoxetine.

2. Procédé selon la revendication 1, caractérisé en ce qu'on effectue l'hydrogénation du composé de formule (II) en présence d'un catalyseur de Pd/C ou Pd-Pt/C.

3. Procédé selon au moins une des revendications 1 et 2, caractérisé en ce qu'on effectue l'hydrogénation en présence d'eau ou d'un solvant organique, par exemple l'acétate d'éthyle.

4. Procédé selon au moins une des revendications 1 à 3, caractérisé en ce qu'on effectue l'hydrogénation à une pression d'hydrogène de 1 à 20 bars, à une température élevée, par exemple à 50°C.

5. Procédé selon au moins une des revendications 1 à 4, caractérisé en ce qu'on effectue l'éthérification du composé III dans un solvant organique, par exemple la N-méthylpyrrolidone, à une température élevée, par exemple à 80°C.
